# EUROPEAN PATENT APPLICATION

(11) **EP 4 541 260 A1**
(43) Date of publication of application: **23.04.2025**
(21) Application number: 23203756.4
(22) Date of filing: 16.10.2023
(51) Int. Cl.: A61B 5/00, A61B 5/372, A61B 5/374

(54) **AN APPARATUS AND A METHOD FOR ACQUIRING ELECTRICAL SIGNALS REPRESENTATIVE OF BRAIN ACTIVITY**

(71) Applicant: Imec VZW, 3001 Leuven (BE)
(72) Inventor: ROTTENBERG, Xavier, 3010 Kessel-Lo (BE); VAN HELLEPUTTE, Nick, 3060 Korbeek-Dijle (BE); PEUMANS, Peter, 1540 Herfelingen (BE)
(74) Representative: AWA Sweden AB

(57) **Abstract**

An apparatus (10) for acquiring electrical signals representative of brain activity comprises:
an ultrasound source (100) configured to emit ultrasound (110) into a brain of a subject (120), forming a focused ultrasound field in a selected region (130) of the brain, wherein the ultrasound field (110) is configured to generate a spatially distributed non-scalar modulation of a naturally occurring electrical signal related to the brain activity of the selected region (130) such that the naturally occurring electrical signal is modulated to combine a frequency of the ultrasound field (110) with the naturally occurring electrical signal, creating a modulated signal, wherein the frequency of the ultrasound field (110) is higher than frequency content of the naturally occurring electrical signal; and
at least one sensor (140, 142) configured to acquire the naturally occurring electrical signal emitted from the selected region (130) of the brain, by sensing the modulated signal.

## Description

### Technical field

The present inventive concept relates to an apparatus and a method for acquiring electrical signals representative of brain activity.

### Background

Given the important and central role of the human brain as the main control center of the human body, in addition to the human brain also being the center of mind and thought, it is of great importance to develop practical and powerful interfaces between the human brain and machines. Existing solutions encompass brain-machine interfaces that targets reading and influencing brain activity. However, current techniques used for that purpose are either enormously expensive and cumbersome or terribly crude delivering poor spatial and temporal resolution as well as depth of sensing. Typical techniques are either functional magnetic resonance imaging (fMRI), which require room-sized equipment, or electroencephalography (EEG), which merge brain activity in one or at best a few monitoring outputs linked to cm³-sized brain areas. Electrodes for EEG acquisition will record the activity of all the neurons in its vicinity. Hence, EEG recording typically suffers from poor spatial resolution.

Poor spatio-temporal resolution, limited depth of access and lack of portability are currently huge limitations for addressing challenges of neuroscience and for treating brain disorders. It is further clearly preventing any practical brain-machine interface development.

A workable brain-machine interface that would provide improved resolution and depth of access in the brain would have tremendous impact on use of brain-machine interaction, as well as on diagnosis and treatment of diseases that originate in the brain, such as dementia and other neurological or neurodegenerative disorders.

### Summary of the invention

It is an objective to, at least partly, mitigate, alleviate, or eliminate one or more of the above-identified deficiencies in the art singly or in any combination.

This and other objectives are at least partly met by the invention as defined in the independent claims. Preferred embodiments are set out in the dependent claims.

According to a first aspect, there is provided an apparatus for acquiring electrical signals representative of brain activity. The apparatus comprises an ultrasound source configured to emit ultrasound into a brain of a subject, forming a focused ultrasound field in a selected region of the brain, wherein the ultrasound field is configured to generate a spatially distributed non-scalar modulation of a naturally occurring electrical signal related to the brain activity of the selected region such that the naturally occurring electrical signal is modulated to combine a frequency of the ultrasound field with the naturally occurring electrical signal, creating a modulated signal, wherein the frequency of the ultrasound field is higher than frequency content of the naturally occurring electrical signal. The apparatus further comprises at least one sensor configured to acquire the naturally occurring electrical signal emitted from the selected region of the brain, by sensing the modulated signal.

Within the context of this disclosure, the phrase "spatially distributed non-scalar modulation" should be construed as the ultrasound field providing a modulation in the selected region of the brain, which has a vectorial or even torsional component, i.e., the modulation is not merely scalar and is spatially distributed. It should be noted that an ultrasound field is associated with a pressure field and a velocity field. The non-scalar modulation involves that the ultrasound field may provide an effect on tissue in the selected region of the brain, wherein the effect is linked to movement of tissue generated locally in the selected region by the focused ultrasound field. The effect on tissue is not purely defined by a pressure in the selected region of the brain caused by the ultrasound field, which would correspond to causing a scalar modulation. Thanks to the ultrasound field affecting movement of tissue, the ultrasound field will also modulate naturally occurring electrical signals propagating in or originating from the selected region of the brain.

Within the context of this disclosure, the phrase "naturally occurring electrical signal" should be construed as any electrical signal that is originating from and propagating in brain tissue. The naturally occurring electrical signal may be based on spontaneous electrical activity in the brain. For instance, the naturally occurring electrical signal may relate to a pattern of activity, which may be referred to as brain waves. The naturally occurring electrical signals may also or alternatively relate to local field potentials or action potentials generated in the brain tissue. The naturally occurring electrical signals may normally exhibit a frequency in a range of 1 Hz-10 kHz, such as 1 - 1000 Hz.

The apparatus according to the first aspect allows for identification and recording of electrical brain signals in a non-invasive way, while still achieving a good spatio-temporal resolution and being able to record signals from deep brain regions.

Deep brain recording with good resolution conventionally requires invasive maneuvers such as an operation, for the purpose of implanting a sensor in the brain. However, according to the first aspect, a focused ultrasound field is used to tag a selected region of the brain, which may enable acquiring of electrical signals in a non-invasive manner.

However, it should be realized that the apparatus need not necessarily be used in a non-invasive manner. It should be realized that the at least one sensor may be placed in an invasive manner for sensing the modulated signal. For instance, the at least one sensor may be arranged under skin of the subject in the epicranium or below the skull. Additionally or alternatively, the ultrasound source may also be placed in an invasive manner.

The focusing of the ultrasound field allows a region to be selected in the brain. This implies that a resolution of the acquired electrical signals may be controlled by the ultrasound field. The ultrasound field may thus facilitate high resolution of acquisition of electrical signals representative of brain activity. For instance, the spatial resolution may be in order of cubic millimeters.

Thanks to the sensing of a modulated electrical signal, the naturally occurring electrical signal may be sensed with a temporal resolution at which electrical activity in the brain takes place (100 microseconds to fractions of seconds), far exceeding the temporal resolution achievable with an MRI (seconds).

The modulated signal is modulated at a high frequency defined by the ultrasound. This implies that the naturally occurring electrical signal is modulated by a frequency to differentiate the naturally occurring electrical signal at the selected region of the brain from electrical signals in other regions of the brain. This implies that the modulated signal may be detected with a high signal-to-noise ratio by the at least one sensor.

The non-scalar modulation provided by the focused ultrasound field causes a direction of vibrations (or movement of tissue) in the selected region of the brain. This implies that the modulated signal may be different in different directions from the selected region. For instance, if a plurality of sensors is used, the sensors may provide non-uniform detection of the modulated signal based on the sensors being arranged in different relations to the selected region. This may be used for improving detection of the modulated signal. This may also be used for providing a degree of freedom based on a direction of vibrations caused by the focused ultrasound field which may be used in coding signals for distinguishing modulated signals from different selected regions of the brain being simultaneously addressed.

Since the frequency of the ultrasound field is higher than frequency content of the naturally occurring electrical signal, the naturally occurring electrical signal will not be perturbed by the ultrasound field. Instead, the modulated signal is formed in the selected region of the brain, wherein the frequency of the ultrasound field is superimposed on the naturally occurring electrical signal.

The ultrasound may have a frequency allowing the ultrasound to propagate deep into the brain with low losses when the ultrasound passes the skull and propagates into the brain. Thus, the ultrasound source may be arranged outside the brain such that a non-invasive manner of providing the ultrasound field in the selected region of the brain is provided.

The ultrasound field may thus be used for selecting a region deep into the brain. Acquisition of naturally occurring electrical signal by sensing of an electrical signal using a sensor arranged outside the brain may be difficult with conventional recording of brain signals, since signals from regions deep into the brain may be weak. However, thanks to the apparatus allowing the modulated signal to be acquired with high signal-to-noise ratio, the apparatus may achieve sensing of signals in regions deep into the brain.

The apparatus may further comprise receiving electronics, where said receiving electronics may comprise a mixer, configured to receive a signal from the at least one sensor and configured to mix the received signal with a signal corresponding to an ultrasound waveform used for emitting ultrasound into the brain of the subject, wherein the mixer is configured to create a combined signal, and a low pass filter configured to filter the combined signal and extract the naturally occurring electrical signal.

The naturally occurring electrical signal is modulated by the frequency of the ultrasound field. The mixer creates a combined signal wherein the naturally occurring electrical signal is again down-modulated to a lower frequency based on being mixed with a signal corresponding to the ultrasound waveform used for emitting ultrasound into the brain. The combined signal may thus be filtered by the low pass filter which extracts the naturally occurring electrical signal. This implies that the naturally occurring electrical signal may be extracted with a high resolution as background noise of low frequencies may be removed by the mixer and the low pass filter.

The mixing carried out by the mixer may be either digital or analogue.

The received signal from the sensor is an electrical signal. The received signal is mixed with another electrical signal which corresponds to the ultrasound waveform. The signal corresponding to the ultrasound waveform may be synchronized to the ultrasound emitted by the ultrasound source or at least have a common base frequency with the ultrasound waveform. The signal corresponding to the ultrasound waveform may be an electrical signal input to an ultrasound transducer for generating the ultrasound waveform.

By isolating only the high-frequency modulated signal and downmodulating the signal back to a baseband by the mixer, the brain activity from only the small, selected region may be recovered while brain activity from other regions may be rejected by the mixer and low pass filter. Ultimately, this implies that a high spatial resolution is provided.

The receiving electronics, comprising the mixer and the low pass filter, may thus be used for increasing the possibility of capturing a signal with high spatial resolution, as the mixer and low pass filter work together to capture the desired signal and filter out the remaining undesired signals and noise. This implies that the desired signal may be extracted with a very high resolution.

The receiving electronics may further comprise an amplifier, connected between the at least one sensor and the mixer and configured to amplify the signal from the at least one sensor before the signal is input to the mixer, wherein the amplifier is adapted to amplify high frequency content corresponding to the frequency of the ultrasound field.

The amplifier may be a different amplifier than normally used when acquiring electrical signals, such as a traditional low frequency amplifier used for EEG. As such, the amplifier may be a high-frequency low noise amplifier, which amplifies the acquired high-frequency signals, which can be differentiated from the otherwise low-frequency electrical signals from the EEG, thus making it easier to acquire the desired signals.

The amplifier may be coupled via capacitive electrodes in order to decrease the prevalence of low-frequency signals in the signal chain.

The amplifier may thus be used to amplify the desired high-frequency signals intended to be fed into the mixer that may create a high-frequency modulated signal. Thus it will be easier to isolate the signal that has been modulated by the focused ultrasound, compared to using a regular amplifier usually installed in an EEG.

The ultrasound source may be configured to emit ultrasound with the ultrasound waveform, wherein the ultrasound waveform comprises a carrier waveform modulated by a characteristic modulation code.

An associated advantage of using a characteristic modulation code is that the naturally occurring electrical signal related to the brain activity of the selected region is further modulated by the characteristic modulation code. This may be used in processing of the sensed modulated signal such that the naturally occurring electrical signal may be extracted with an even higher resolution thanks to the modulation code.

The characteristic modulation code may be provided as amplitude and/or phase modulation of the carrier waveform.

This may be a suitable manner of providing a modulation code of the emitted ultrasound.

The modulation code may comprise multiple coding bits. Each coding bit may last at least one period of the carrier waveform. The coding bit may be defined by a level of the amplitude and/or phase modulation or by amplitude and/or phase modulation being on or off, in order to define a value of the coding bit. The modulation code may repeat at a frequency which is at least higher than a frequency of the naturally occurring electrical signal, such that the modulation code may be utilized in high resolution extraction of the naturally occurring electrical signal from the modulated signal.

The ultrasound source may be configured to emit ultrasound forming a plurality of ultrasound fields, wherein each ultrasound field has a unique characteristic modulation code.

An associated advantage with the ultrasound having a plurality of fields, each with a unique characteristic modulation code is that it enables the possibility to provide modulation of naturally occurring electrical signals in a plurality of different brain regions at the same time.

The unique characteristic modulation codes enable naturally occurring electrical signals from different selected regions to be differentiated from each other.

The apparatus may thus be able to increase the amount of possible measurement points of the brain of the patient. Put differently, with an increased number of possible brain regions to measure simultaneously, it would be possible to record and analyze a wider range of brain regions and/or to faster record signals from a multitude of measurement points. This may allow detecting of naturally occurring electrical signals from a plurality of measurement points with a high temporal resolution.

The ultrasound source may comprise a plurality of ultrasound transducers configured to be controlled to form a phased array.

An associated advantage of this is that a phased array facilitates controlling a direction of the ultrasound field and a depth of the selected region in the brain. The beam-forming properties of a phased array may be used for controlling a location of the selected region in which the focused ultrasound field is formed.

The ultrasound source may thus be fast and precise in localizing the selected region of the brain that is targeted by the focused ultrasound field.

The ultrasound source may be configured to emit the ultrasound field exhibiting a frequency in a range of 500 kHz - 2 MHz.

An associated advantage of this is that this frequency spectrum enables the ultrasound field to pass the skull and reach deep regions in the brain.

The ultrasound source thus may have an ability to easily penetrate the bone of the skull and reach the desired depth of the brain without a significant drop in ultrasound energy. Thus, the specified frequency range may provide a strong modulated signal such that a high resolution of the acquired signal may be provided.

The apparatus may comprise a plurality of sensors, each configured to acquire the naturally occurring electrical signal emitted from the selected region of the brain, by sensing the modulated signal, wherein the apparatus may be configured to cross-correlate modulated signals acquired by the plurality of sensors.

The apparatus thus may collect the naturally occurring electrical signals from a plurality of positions. At each of the positions, the naturally occurring electrical signal from the same selected region of the brain may be sensed. However, each sensor may acquire the same naturally occurring electrical signal with a different noise. Using cross-correlating of the modulated signals acquired by the plurality of sensors, it may therefore be possible to suppress the uncorrelated background noise and increasingly isolate any signal of interest, such that signal-to-noise ratio is increased.

At least one sensor may comprise an optical sensor.

The optical sensor may comprise a light source configured to transmit light. The transmitted light may interact with the modulated signal allowing the optical sensor to sense light that have interacted with the modulated signal for sensing the modulated signal.

The optical sensor may be coupled to optical read-out for forming an electric signal based on the light sensed by the optical sensor. The optical sensor may be combined with receiving electronics for mixing the received signal with a signal corresponding to the ultrasound waveform and a low pass filter for extracting the naturally occurring electrical signal. The optical sensor may be used for detecting ultrasound being modulated with a characteristic modulation code.

At least one sensor may comprise an electrical sensor.

The electrical sensor may thus be configured to detect the electrical modulated signal. The electrical sensor may be coupled to read-out circuitry for reading out an electrical signal from the electrical sensor.

The at least one sensor may comprise a magnetic sensor.

The magnetic sensor may be configured to detect a magnetic field formed by the modulated signal. The optical sensor may be coupled to magnetic read-out for forming an electric signal based on the magnetic field sensed by the magnetic sensor.

The at least one sensor may be an ultrasound sensor. The ultrasound sensor may comprise an ultrasound transducer for converting the modulated signal to an ultrasound signal, which may then be sensed.

These are suitable sensors for sensing the modulated signal.

The electrical sensor may be configured to provide a capacitive coupling between the body of the subject and an electrode of the electrical sensor.

An associated advantage of this is that a capacitive coupling between the body of the subject and an electrode, rather than a resistive coupling, creates a high-pass filter. Thus, the electric signals of higher frequencies are captured rather than the normal signals usually acquired for sensing brain signals. This implies that direct current (DC) offsets which may otherwise make it difficult to extract the naturally occurring electrical signal may be completely avoided in the sensed signal.

The electrical sensor may thus minimize the undesired recording of noise, electrical signals not stemming from brain activity or brain signals that are not frequency-modulated into a higher frequency baseband.

In addition, when low frequency signals are to be acquired, electrodes usually need to be provided with gel to achieve low impedance for acquiring the low frequency signals. However, thanks to using a capacitive coupling, a good contact impedance may be provided between the body of the subject and the electrode for the high frequencies of interest. This implies that it may not be necessary to use any gel for providing good contact between the body of the subject and the electrode.

According to a second aspect, there is provided a method for acquiring electrical signals representative of brain activity. The method comprising emitting, from an ultrasound source, ultrasound into a brain of a subject to form a focused ultrasound field directed towards a selected region of the brain, wherein the ultrasound field is configured to generate, a spatially distributed non-scalar modulation of a naturally occurring electrical signal related to the brain activity of the selected region such that the naturally occurring electrical signal is modulated to combine a frequency of the ultrasound field with the naturally occurring electrical signal, whereby a modulated signal is created, wherein the frequency of the ultrasound field is higher than frequency content of the naturally occurring electrical signal. The method further comprising acquiring the naturally occurring electrical signal emitted from the selected region of the brain, by using at least one sensor configured to sense the modulated signal.

The method according to the second aspect allows for identification and recording of electrical brain signals in a non-invasive way, while still achieving a good spatio-temporal resolution and being able to record signals from deep brain regions.

The method may further comprise mixing, by a mixer, a signal received from the at least one sensor with a signal corresponding to an ultrasound waveform used for emitting ultrasound into the brain of the subject, whereby a combined signal is created, and filtering, with a low pass filter, the combined signal such that the naturally occurring electrical signal is extracted.

This implies that the naturally occurring electrical signal may be extracted with a high resolution as background noise of low frequencies may be removed by the mixer and the low pass filter.

Emitting may comprise, emitting ultrasound with the ultrasound waveform, wherein the ultrasound waveform may comprise a carrier waveform modulated by a characteristic modulation code.

The modulation code may be used in processing of the sensed modulated signal such that the naturally occurring electrical signal may be extracted with an even higher resolution thanks to the modulation code.

The above-mentioned features of the first aspect, when applicable, apply to this second aspect as well. In order to avoid undue repetition, reference is made to the above.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1 illustrates an apparatus for acquiring electrical signals representative of brain activity according to an embodiment.
Fig. 2 illustrates receiving electronics of the apparatus of Fig. 1.
Fig. 3 illustrates an ultrasound source of the apparatus according to an embodiment.
Fig. 4 illustrates an ultrasound waveform.
Fig. 5 is a flow chart of a method for acquiring electrical signals representative of brain activity.

### Detailed description

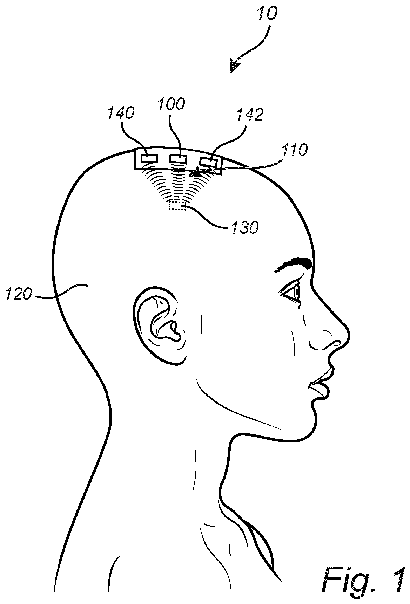

In the following, an apparatus 10 for acquiring electrical signals representative of brain activity will be described in connection with Figs 1-4.

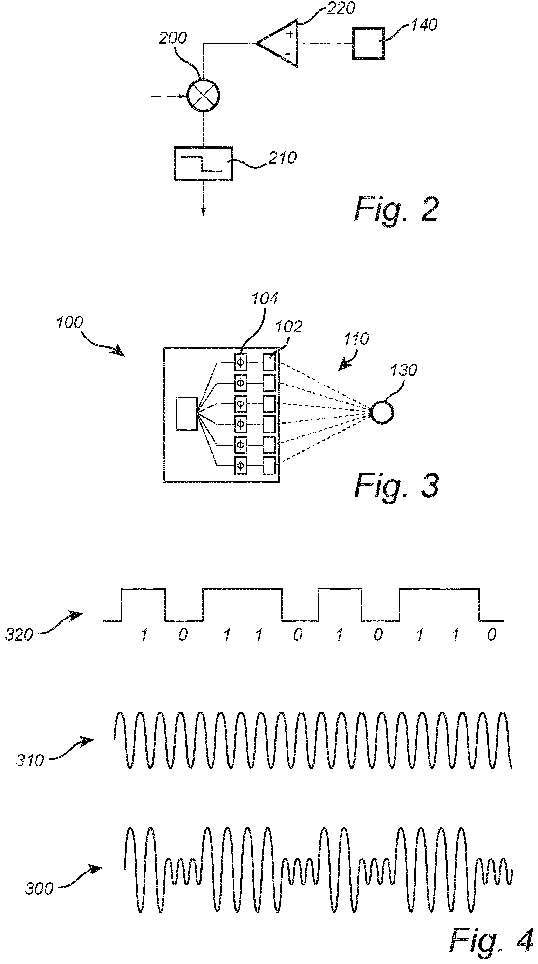

The apparatus 10 comprises an ultrasound source 100 and a sensor 140, as illustrated in Fig. 1. The apparatus 10 may optionally comprise a plurality of sensors and an additional sensor 142 is illustrated in Fig. 1. Each sensor 140, 142 may function in the same manner. For brevity, only the sensor 140 will be initially discussed. The apparatus 10 may also comprise components of receiving electronics 20, such as a mixer 200, a low pass filter 210 and an amplifier 220, as illustrated in Fig. 2.

As illustrated in Fig. 1, the ultrasound source 100 is configured to emit ultrasound 110 into a brain of a subject 120, forming a focused ultrasound field in a selected region 130 of the brain. The ultrasound source 100 is thus configured to be able to form a focused ultrasound field in the selected region 130. This may be achieved by the ultrasound source 100 comprising a plurality of transducers, which may generate a focused ultrasound field based on interference between ultrasound output by different transducers. However, the ultrasound source 100 may alternatively output an ultrasound field which is converging to form a focused ultrasound field in the selected region 130.

The ultrasound field 110 is configured to generate a spatially distributed non-scalar modulation of a naturally occurring electrical signal related to the brain activity of the selected region 130. Thus, the naturally occurring electrical signal is modulated to combine a frequency of the ultrasound field 110 with the naturally occurring electrical signal, creating a modulated signal, wherein the frequency of the ultrasound field 110 is higher than frequency content of the naturally occurring electrical signal.

The apparatus 10 further comprises the sensor 140 configured to acquire the naturally occurring electrical signal emitted from the selected region of the brain, by sensing the modulated signal.

The focusing of the ultrasound field enables a selected region 130 of the brain to be tagged. Thus, the region 130 may be selected in the brain of the subject 120 such that the apparatus 10 may acquire signals that can be associated with the selected region 130. This implies that a spatial resolution of the acquired electrical signals may be controlled by the focusing of the ultrasound field 110. The ultrasound field 110 may thus facilitate high resolution of acquisition of electrical signals representative of brain activity by accurately defining the selected region 130. For instance, the spatial resolution may be in order of cubic millimeters.

It should be realized that the focused ultrasound field 110 may provide a non-scalar modulation controlling a pressure field and a velocity field of the ultrasound in the selected region 130. Thus, the ultrasound field 110 may provide a high intensity of the pressure field in the selected region 130 such that the intensity of the pressure field of the ultrasound field 110 outside the selected region 130 is much smaller than the intensity of the pressure field of the ultrasound field 110 in the selected region 130. In addition, the velocity field in the selected region 130 may have a selected direction so as to provide a direction of the ultrasound field 110 within the selected region. The ultrasound field 110 may thus be provided such that any modulation of naturally occurring electrical signals outside the selected region 130 may have such a low intensity so as to not affect detection of the naturally occurring electrical signals.

The naturally occurring signal is modulated at a high frequency defined by the ultrasound 110. This implies that the modulated signal has a frequency differentiating the naturally occurring electrical signal at the selected region 130 of the brain from electrical signals in other regions of the brain. This implies that the modulated signal may be detected with a high signal-to-noise ratio by the sensor 140.

The naturally occurring electrical signal may be any electrical signal that is originating from and propagating in brain tissue. The naturally occurring electrical signal may be based on spontaneous electrical activity in the brain. For instance, the naturally occurring electrical signal may relate to a pattern of activity, which may be referred to as brain waves, which may normally exhibit a frequency in a range of 1-30 Hz. The brain waves may be any neural oscillations originating from the brain of the subject 120, for example alpha, beta, gamma, delta, or theta waves. However, the naturally occurring electrical signals may also or alternatively relate to local field potentials or action potentials generated in the brain tissue. The naturally occurring electrical signals may exhibit a frequency in a range of 1 Hz-10 kHz, such as 1 - 1000 Hz.

Since the frequency of the ultrasound field 110 is higher than frequency content of the naturally occurring electrical signal, the naturally occurring electrical signal S2 will not be perturbed by the ultrasound field 110. Instead, the modulated signal is formed in the selected region 130 of the brain, wherein the frequency of the ultrasound field 110 is superimposed on the naturally occurring electrical signal S3.

The ultrasound field 110 provides a spatially distributed non-scalar modulation of the naturally occurring electrical signal meaning that the ultrasound field 110 providing a modulation in the selected region 130 of the brain has a vectorial or even torsional component, i.e., the modulation is not merely scalar and is spatially distributed.

Thus, the ultrasound field 110 may provide an effect on tissue in the selected region 130 of the brain, wherein the effect is linked to movement of tissue generated locally in the selected region 130 by the focused ultrasound field 110.

The ultrasound source 100 may be configured to emit the ultrasound field 110 exhibiting a frequency in a range of 500 kHz - 2 MHz. This implies that the frequency of the ultrasound field 110 differs substantially from the frequency content of the naturally occurring electrical signal. This also implies that the ultrasound field 110 may be adapted to propagate deep into the brain with low losses when the ultrasound field 110 passes the skull and propagates into the brain.

The ultrasound field 110 may thus have an ability to easily penetrate the bone of the skull and provide deep brain sensing by modulating the naturally occurring electrical signal in a selected region 130 deep into the brain.

As shown in Fig. 1, the ultrasound source 100 may be arranged outside the brain such that a non-invasive manner of providing the ultrasound field 110 in the selected region 130 of the brain is provided. However, it should be realized that the ultrasound source 100 and/or the sensor 140 may alternatively be arranged in an invasive manner such as arranged under skin of the subject in the epicranium or below the skull.

The sensor 140 may be arranged relatively close to the ultrasound source 100 outside the brain. Thus, the sensor 140 may be arranged close to the selected region 130 to allow sensing a strong modulated signal as the modulated signal need not propagate a long distance between the selected region 130 and the sensor 140. The ultrasound source 100 and the sensor 140 may even be arranged in a common physical unit, such as a patch, so that the ultrasound source 100 and the sensor 140 may be simultaneously arranged in relation to the subject 120, when the apparatus 10 is arranged in relation to the subject 120.

However, it should be realized that the sensor 140 may be arranged relatively far apart from the ultrasound source 100, and that the sensor 140 and the ultrasound source 100 are arranged in different physical units.

The sensor 140 may be configured to detect electrical signals propagating in the brain of the subject 120. The sensor 140 may thus sense the modulated signal. However, it should be realized that the sensor 140 may alternatively be an optical sensor, a magnetic sensor, or an ultrasound sensor. Regardless, the sensor 140 may be coupled to read-out components for forming an electrical signal based on the signal sensed by the sensor.

In case, an optical sensor is used, the optical sensor may comprise a light source configured to transmit light. The transmitted light may interact with the modulated signal allowing the optical sensor to sense light that have interacted with the modulated signal for sensing the modulated signal.

The light source may be configured to transmit coherent or noncoherent light. The light source may transmit light in for instance visible region, near-infrared range or infrared range of the electro-magnetic spectrum. The optical sensor may use a single light detector or an array of light detectors for sensing the modulated signal.

Below, the processing of electrical signals will be described, which may mainly apply to the signal sensed by an electrical sensor. However, as understood by the person skilled in the art, the processing of signals may be adapted to fit a signal detected by a different kind of sensor.

As illustrated in Fig. 2, after the modulated signal have been acquired and received by the sensor 140, the mixer 200 may be configured to mix the received signal with a signal corresponding to an ultrasound waveform 300 (discussed further below in relation to Fig. 4) used for emitting ultrasound into the brain of the subject 120. For instance, the mixer 200 may be configured to receive an electrical control signal for generating the ultrasound field 110, which control signal defines the ultrasound waveform 300. The mixer 200 is further configured to receive the modulated signal acquired by the sensor 140, and the mixer 200 may be configured to create a combined signal based on the modulated signal and the signal corresponding to the ultrasound waveform 300.

The combined signal may then be passed through the low pass filter 210, which is configured to filter the combined signal and extract the naturally occurring electrical signal.

The naturally occurring electrical signal is modulated by the frequency of the ultrasound field 110, as described above. This implies that the naturally occurring electrical signal from the selected region 130 is up-modulated into a frequency band based on the frequency of the ultrasound field 110. The mixer 140 creates a combined signal, wherein the naturally occurring electrical signal is again down-modulated to a lower frequency band (the original frequency of the naturally occurring electrical signal) based on being mixed with the signal corresponding to the ultrasound waveform 300 used for emitting ultrasound into the brain. The combined signal may thus be filtered by the low pass filter 210 which extracts the naturally occurring electrical signal. This implies that the naturally occurring electrical signal may be extracted with a high resolution as background noise of low frequencies may be removed by the mixer 200 and the low pass filter 210. In fact, the modulated signal may be very weak and even below a noise floor but thanks to mixing the modulated signal with the signal corresponding to the ultrasound waveform, it is possible to sense brain activity.

The mixing carried out by the mixer 200 may be performed either in digital or analog domain. The mixer 200 may use heterodyne mixing.

By isolating only the high-frequency modulated signal and downmodulating the signal back to a baseband by the mixer 200, the brain activity from only the selected region 130 may be recovered while brain activity from other regions may be rejected by the mixer 200 and the low pass filter 210. Ultimately, this implies that a high spatial resolution is provided.

In addition to the mixer 200 and the low pass filter 210, as illustrated in Fig. 2, the amplifier 220 may be connected between the sensor 140 and the mixer 200. The amplifier 220 may be configured to amplify the signal from the sensor 140 before the signal is input to the mixer 200, wherein the amplifier 220 is adapted to amplify high frequency content corresponding to the frequency of the ultrasound field 110.

The amplifier 220 may be a high-frequency low noise amplifier, which amplifies the acquired high-frequency signals. Thus, the amplifier 220 may be configured to amplify the modulated signal received by the sensor 140 without degrading a signal-to-noise ratio.

In an embodiment where the sensor 140 is an electrical sensor, the sensor 140 may be configured to provide a capacitive coupling between the body of the subject 120 and an electrode of the electrical sensor 140. Use of a capacitive coupling between the body of the subject 120 and the electrode may create a high-pass filter. Thus, low-frequency signals are not captured by the electrical sensor 140. This implies that, for instance, naturally occurring electrical signals which have not been modulated will not be detected by the sensor 140 and will therefore not affect detection of the signal of interest, namely the naturally occurring electrical signal from the selected region 130. Also, the use of a capacitively coupled sensor 140 implies that direct current (DC) offsets, which may otherwise make it difficult to extract the naturally occurring electrical signal, may be completely removed in the sensed signal.

The electrical sensor may thus minimize the undesired recording of noise and electrical signals stemming from brain activity outside the selected region 130.

Since the sensor 140 may provide a high-pass filter function, the signal from the sensor 140 may not include low frequency signals. This also implies that the amplifier 220 does not receive low frequency signals and that prevalence of low-frequency signals in the signal chain is decreased.

The amplifier 220 may thus be used to amplify the desired high-frequency signals intended to be fed into the mixer that may create a high-frequency modulated signal. This facilitates isolating the signal that has been modulated by the focused ultrasound.

As illustrated in Fig. 3, the ultrasound source 100 may comprise a plurality of ultrasound transducers 102 configured to be controlled to form a phased array. This may be achieved by each transducer 102 being associated with a delay 104, which may define a phase delay of the transducer 102 such that different phase delays may be provided for the ultrasound output by each of the transducers 102. An associated advantage of this is that the phased array facilitates controlling a direction of the ultrasound field 110 and a depth of the selected region 130 in the brain. The beam-forming properties of the phased array may be used for controlling a location of the selected region 130 in which the focused ultrasound field is formed.

The ultrasound source 100 may thus be fast and precise in localizing the selected region 130 of the brain that is targeted by the focused ultrasound field 110.

It should be realized that the ultrasound source 100 may be controlled for enabling detection of naturally occurring electrical signals from a plurality of selected regions. Using a phased array, the ultrasound source 100 may be easily controlled so as to sequentially provide a focused ultrasound field 110 in different selected regions 130. Thus, naturally occurring electrical signals may be sequentially detected from the different selected regions 130. This may be used for scanning a part of or even the entire brain of the subject 120.

The ultrasound source 100 forming a phased array facilitates controlling a location of the selected region 130 of the brain. However, it should be realized that the ultrasound source 100 need not necessarily form a phased array in order for allowing the location of the selected region 130 to be controlled. Rather, the ultrasound source 100 may for instance comprise a movable transducer for changing a direction of the focused ultrasound field 110.

The apparatus 10 may comprise a plurality of sensors 140, 142. Each of the plurality of sensors 140, 142 may be configured to acquire the naturally occurring electrical signal emitted from the selected region 130 by sensing the modulated signal. The plurality of sensors 140, 142 may be arranged in different locations. This implies that the plurality of sensors 140, 142 may sense the modulated signal at different locations.

The apparatus 10 thus may collect the naturally occurring electrical signals from a plurality of locations. At each of the locations, the naturally occurring electrical signal from the same selected region 130 of the brain may be sensed. However, each sensor 140, 142 may acquire the same naturally occurring electrical signal with a different noise. Using cross-correlating of the modulated signals acquired by the plurality of sensors 140, 142, it may therefore be possible to suppress the uncorrelated background noise and increasingly isolate any signal of interest, such that signal-to-noise ratio of detecting the naturally occurring electrical signal is increased.

As illustrated in Fig. 4, the ultrasound field 110 may comprise an ultrasound waveform 300, wherein the ultrasound waveform 300 may comprise a carrier waveform 310 modulated by a characteristic modulation code 320. By using the characteristic modulation code 320 the naturally occurring electrical signal related to the brain activity of the selected region 130 may be further modulated by the characteristic modulation code 320. This may be used in processing of the sensed modulated signal such that the naturally occurring electrical signal may be extracted with an even higher resolution thanks to the modulation code 320. The characteristic modulation code 320 may be provided as amplitude modulation of the carrier waveform 310 as indicated in Fig. 4. This may be a suitable manner of providing a modulation code of the emitted ultrasound 110. However, it should be realized that other types of modulation may be provided. For instance, the characteristic modulation code may be provided as on-off keying modulation, binary phase-shift keying modulation, frequency modulation or phase modulation, or even higher order modulation, such as quadrature amplitude modulation.

The modulation code 320 may comprise multiple coding bits. Each coding bit may last at least one period of the carrier waveform 310. The coding bit may for instance be defined by a level of the amplitude and/or phase modulation or by amplitude and/or phase modulation being on or off, in order to define a value of the coding bit. The modulation code 320 may have a length such that the modulation code 320 is repeated at a frequency which is at least higher than a frequency of the naturally occurring electrical signal. Thus, the modulation code may be utilized in high resolution extraction of the naturally occurring electrical signal S2 from the modulated signal. Fig. 3 shows two repetitions of a modulation code being 5 bits long, but it should be realized that this is just an illustrative example and longer or shorter codes may be used.

However, it should be realized that the ultrasound waveform 300 need not necessarily include a modulation code 320. Rather, the ultrasound waveform 300 may be based purely on a carrier waveform having an ultrasound frequency.

The ultrasound source 100 may comprise a plurality of ultrasound units for outputting ultrasound. The ultrasound units may be arranged at different locations in relation to the brain of the subject 120. The plurality of ultrasound units may be configured to simultaneously emit ultrasound, wherein the ultrasound from each ultrasound unit forms a focused ultrasound field 110 in a unique selected region 130. Thus, a plurality of selected regions 130 may simultaneously receive focused ultrasound fields 110 so as to enable simultaneous detection of naturally occurring electrical signals in a plurality of selected regions 130 of the brain.

Each ultrasound field 110 may have a unique characteristic modulation code 320. This enables detection of the naturally occurring electrical signals in a plurality of different selected regions 130 at the same time.

The unique characteristic modulation codes 320 enable naturally occurring electrical signals from different selected regions 110 to be differentiated from each other. The modulation code 320 may use orthogonal spreading codes for allowing forming of unique characteristic modulation codes 320 for the different selected regions 130.

Each sensor 140, 142 may be associated with a plurality of mixers 200. Thus, the signal sensed by the sensor 140, 142 may be provided to a plurality of channels, each having a separate mixer 200. The mixer in each channel may mix the sensed modulated signal with a unique ultrasound waveform 300. Thus, in each channel, the naturally occurring electrical signal from one of the selected regions 130 may be extracted. This also implies that the signals from the selected regions 130 may be processed in parallel by a plurality of mixers in a plurality of channels.

The apparatus 10 may thus be able to increase the amount of possible measurement points of the brain of the patient. Put differently, with an increased number of possible brain regions to measure simultaneously, it would be possible to record and analyze a wider range of brain regions and/or to faster record signals from a multitude of measurement points. This may allow detecting of naturally occurring electrical signals from a plurality of measurement points with a high temporal resolution.

The apparatus 10 may comprise a housing in which all components of the apparatus 10 are arranged. Thus, the ultrasound source 100 (possibly having a plurality of ultrasound units) and the at least one sensor 140, 142 may be arranged in a single physical housing. This may imply that the physical relation between the ultrasound source 100 and the sensor(s) 140, 142 may be accurately defined within the housing. This also implies that the apparatus 10 may be arranged in relation to the brain of a subject 120 in a simple manner.

The housing may be arranged in form of a patch carrying the ultrasound source 100 and the at least one sensor 140, 142. The patch may facilitate attachment of the apparatus 10 externally on a scalp of the subject 120. The housing may also be shaped so as to fit around a part of the scalp, such as having a rounded form. In an embodiment, the housing may be in the form of a cap that may be worn on the scalp.

However, it should be realized that the ultrasound source 100 and the at least one sensor 140, 142 may alternatively be arranged in separate physical units that are separately attached to the scalp of the subject 120. This may provide a higher degree of freedom in placing the ultrasound source 100 and the sensor(s) 140, 142 in relation to each other.

The ultrasound source 100 and the at least one sensor 140, 142 may still be connected through wires, directly to each other or via further units. For instance, the ultrasound source 100 and the at least one sensor 140, 142 may each be connected by wire to a central unit. This may for instance ensure that the signal corresponding to the ultrasound waveform may be mixed with the signal sensed by the at least one sensor 140, 142.

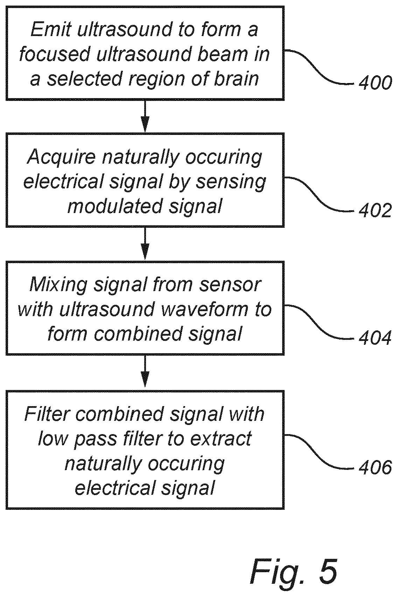

Referring now to Fig. 5, a method for acquiring electrical signals representative of brain activity is presented. The method 50 may for example, be implemented in an ultrasonic transducer, used in a device for medical ultrasonic imaging technique.

The method comprises: emitting 400, from an ultrasound source, ultrasound into a brain of a subject to form a focused ultrasound field directed towards a selected region of the brain. As discussed above, the ultrasound field is configured to generate a spatially distributed non-scalar modulation of a naturally occurring electrical signal related to the brain activity of the selected region such that the naturally occurring electrical signal is modulated to combine a frequency of the ultrasound field with the naturally occurring electrical signal, whereby a modulated signal is created. The frequency of the ultrasound field is higher than frequency content of the naturally occurring electrical signal. The emitting 400 may comprise, emitting ultrasound with an ultrasound waveform, wherein the ultrasound waveform may comprise a carrier waveform modulated by a characteristic modulation code. The modulation code may be used in processing of the sensed modulated signal such that the naturally occurring electrical signal may be extracted with an even higher resolution thanks to the modulation code.

The method further comprises acquiring 402 the naturally occurring electrical signal emitted from the selected region 130 of the brain, by using at least one sensor 140 configured to sense the modulated signal.

Furthermore, the method may comprise the mixing 404, by a mixer, a signal received from the at least one sensor with a signal corresponding to the ultrasound waveform used for emitting ultrasound into the brain of the subject, whereby a combined signal is created. This is followed by filtering 406, with a low pass filter, the combined signal such that the naturally occurring electrical signal is extracted. These additional steps may allow for extracting the naturally occurring electrical signal with a high resolution as background noise may be removed by the mixer 200 and the low pass filter 210.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. An apparatus (10) for acquiring electrical signals representative of brain activity, said apparatus comprising:
an ultrasound source (100) configured to emit ultrasound (110) into a brain of a subject (120), forming a focused ultrasound field in a selected region (130) of the brain, wherein the ultrasound field (110) is configured to generate a spatially distributed non-scalar modulation of a naturally occurring electrical signal related to the brain activity of the selected region (130) such that the naturally occurring electrical signal is modulated to combine a frequency of the ultrasound field (110) with the naturally occurring electrical signal, creating a modulated signal, wherein the frequency of the ultrasound field (110) is higher than frequency content of the naturally occurring electrical signal; and
at least one sensor (140, 142) configured to acquire the naturally occurring electrical signal emitted from the selected region (130) of the brain, by sensing the modulated signal.

2. The apparatus (10) according to claim 1, further comprising receiving electronics (20), said receiving electronics comprising:
a mixer (200), configured to receive a signal from the at least one sensor (140, 142) and configured to mix the received signal with a signal corresponding to an ultrasound waveform (300) used for emitting ultrasound (110) into the brain of the subject (120), wherein the mixer (200) is configured to create a combined signal;
a low pass filter (210) configured to filter the combined signal and extract the naturally occurring electrical signal.

3. The apparatus (10) according to claim 2, wherein the receiving electronics (20) further comprises an amplifier (220), connected between the at least one sensor (140, 142) and the mixer (200) and configured to amplify the signal from the at least one sensor (140, 142) before the signal is input to the mixer (200), wherein the amplifier (220) is adapted to amplify high frequency content corresponding to the frequency of the ultrasound field (110).

4. The apparatus (10) according to claim 2 or 3, wherein the ultrasound source (100) is configured to emit ultrasound (110) with the ultrasound waveform (300), wherein the ultrasound waveform (300) comprises a carrier waveform (310) modulated by a characteristic modulation code (320).

5. The apparatus (10) according to claim 4, wherein the characteristic modulation code (320) may be provided as amplitude and/or phase modulation of the carrier waveform (310).

6. The apparatus (10) according to claim 4 or 5, wherein the ultrasound source (100) is configured to emit ultrasound (110) forming a plurality of ultrasound fields, wherein each ultrasound field has a unique characteristic modulation code (320).

7. The apparatus (10) according to any one of the preceding claims, wherein the ultrasound source (100) comprises a plurality of ultrasound transducers (102) configured to be controlled to form a phased array.

8. The apparatus (10) according to any one of the preceding claims, wherein the ultrasound source (100) is configured to emit the ultrasound field (110) exhibiting a frequency in a range of 500 kHz - 2 MHz.

9. The apparatus (10) according to any one of the preceding claims, wherein the apparatus (10) comprises a plurality of sensors (140, 142), each configured to acquire the naturally occurring electrical signal emitted from the selected region (130) of the brain, by sensing the modulated signal, wherein the apparatus (10) is configured to cross-correlate modulated signals acquired by the plurality of sensors (140, 142).

10. The apparatus (10) according to claim 1, wherein the at least one sensor (140, 142) comprises an optical sensor.

11. The apparatus (10) according to any one of the preceding claims, wherein the at least one sensor (140, 142) comprises an electrical sensor.

12. The apparatus (10) according to claim 11, wherein the electrical sensor is configured to provide a capacitive coupling between the body of the subject and an electrode of the electrical sensor.

13. A method for acquiring electrical signals representative of brain activity, said method comprising;
emitting (400), from an ultrasound source, ultrasound into a brain of a subject to form a focused ultrasound field directed towards a selected region of the brain, wherein the ultrasound field is configured to generate, a spatially distributed non-scalar modulation of a naturally occurring electrical signal related to the brain activity of the selected region such that the naturally occurring electrical signal is modulated to combine a frequency of the ultrasound field with the naturally occurring electrical signal, whereby a modulated signal is created, wherein the frequency of the ultrasound field is higher than frequency content of the naturally occurring electrical signal; and
acquiring (402) the naturally occurring electrical signal emitted from the selected region of the brain, by using at least one sensor configured to sense the modulated signal.

14. The method according to claim 13, wherein the method further comprises:
mixing (404), by a mixer, a signal received from the at least one sensor with a signal corresponding to an ultrasound waveform used for emitting ultrasound into the brain of the subject, whereby a combined signal is created;
filtering (406), with a low pass filter, the combined signal such that the naturally occurring electrical signal is extracted.

15. The method according to claim 14, wherein said emitting (400) comprises emitting ultrasound with the ultrasound waveform, wherein the ultrasound waveform comprises a carrier waveform modulated by a characteristic modulation code.
